# EUROPEAN PATENT SPECIFICATION

(11) **EP 1 640 030 B1**
(45) Date of publication and mention of the grant of the patent: **26.01.2011**
(21) Application number: 03738545.7
(22) Date of filing: 27.06.2003
(51) Int. Cl.: A61M 5/50

(54) **DISPOSABLE INJECTOR**
EINWEG-INJEKTOR
INJECTEUR JETABLE

(43) Date of publication of application: 29.03.2006
(73) Proprietor: Mori, Akihide, Osaka-shi, Osaka 537-0012 (JP)
(72) Inventor: MORI, Akihide, Osaka-shi, Osaka 537-0012 (JP); SAKAI, Shin, Ashiya-shi, Hyogo 659-0013 (JP)
(74) Representative: Hering, Hartmut
(86) International application number: PCT/JP2003/008180
(87) International publication number: WO 2005/000383

(56) References cited:
- JP-A- 3 505 686
- JP-A- 8 229 121
- JP-A- 9 504 457
- JP-A- 10 165 500
- JP-A- 52 131 685
- JP-A- 2001 104 482
- JP-A- 2001 516 235
- JP-U- 56 125 256

## Description

### Technical Field

The present invention relates to disposable injector syringes and, in particular, to a disposable injector syringe that helps users avoid mixing up one syringe with another with each syringe prefilled with a liquid medicine, and helps the users avoid reusing the used syringe with a needle stored in a piston after use so that the syringe is easily and safely disposed.

### Background Art

Syringes currently used in medical fields are typically made of plastic, such as acrylic resins. Most of syringe bodies are made of glass. The syringes together with the needles thereof are sterilized by boiling for reuse. Before use, a user sucks a target liquid medicine into the syringe from an ampule each time. The user needs to check and memorize the name of the liquid medicine at the suction of a liquid medicine. After the syringe is filled with the liquid medicine, the user may be busy enough to mix up one name with another, or after a long period of time, the user may forget the name. Healthcare professionals must carefully handle and manage the syringes. Japanese Open Gazette No. 9-135900 discloses prefilling of a liquid medicine sterilized plastic injector syringe that includes a syringe body filled beforehand with a liquid medicine to control time to transfer the liquid medicine from an ampule to the syringe and the occurrence of errors during the liquid medicine transfer.

Disposable syringes have the problems of disposing needles after use. If a disposable syringe and a needle thereof are disposed, the needle tip may injure a human body. Japanese Open Gazette No. 11-342200 discloses a disposable syringe that allows a needle to be stored into a syringe body.

In the disposable syringe disclosed in Japanese Open Gazette No. 11-342200, a needle is connected to a barrel via a foot. When the foot is connected to a piston, the needle is drawn into the barrel for storage. The foot and the piston must have a mutual engagement mechanism. The structure of the syringe becomes complex.

A syringe having a syringe body for prefilling a liquid medicine has been known. Japanese Open Gazette No. 9-135900 discloses the manufacturing method of the syringe. The syringe holds a liquid medicine in a barrel thereof with a piston sealing the open end of the barrel. A handle needs to be attached to the piston. The needle cannot be stored in the piston before use.

Further documents relating to some features of a syringe according to the present invention are JP-A-9 504 457, JP-A-3 505 686, JP 2001 516235 A, and JP 56 125 256 A. These documents relate to features of the claims 4, 5, 6 and 7, respectively.

The closest prior art document FR 2 347 056 A discloses a disposable injector syringe as desribed in the preamble of claim 1.

It is an object of the present invention to provide a disposable injector syringe that helps users avoid mixing up one syringe with another with each syringe prefilled with a liquid medicine, and helps the users avoid reusing the used syringe with a needle stored in a piston after use so that the syringe is easily and safely disposed. Further, a leakage of the liquid medicine shall be prevented.

### Disclosure of the Invention

A disposable injector syringe, as disclosed in FR 2 347 056 A comprises a syringe body, the syringe body including a cylindrical barrel having a needle mounting portion on one end thereof, and an opening portion, on the other end thereof, for receiving a piston, a needle to be mounted on the needle mounting portion, the piston to be inserted through the syringe body through the opening portion, and restricting means for restricting a movement of the piston, the restricting means restricting the movement of the piston so that the cylindrical barrel is prefilled with a liquid medicine and then closed by the piston.

Since the disposable injector syringe is prefilled with the liquid medicine, time required to transfer the liquid medicine from an ampule is eliminated. In emergency situations, the disposable injector syringe can be quickly handled, and the mixed-up of liquid medicines is prevented from occurring.

According to the present invention as defined in the characterizing portion of claim 1, the piston movement restricting means comprises a bellows, and a fixing sheet, the bellows being composed of a circumferential portion having a predetermined width and a trough portion, the two portions alternating with each other, the bellows covering a piston rod of the piston, and the fixing sheet being glued onto the circumferential portion of the bellows.

With the fixing sheet glued, the shrinking of the bellows is restricted. The piston remains stationary, preventing leakage of the liquid medicine. By simply peeling of the fixing sheet, the bellows immediately becomes shrinkable, thereby pushing the piston to inject the liquid medicine.

Further, the piston has a stability guide ring supported on the piston. This guide ring possibly supports a piston rod in order to slide smoothly.

The piston rod includes a hollow portion, an opening storage portion communicating with the hollow portion, and a cap closing the opening storage portion. The needle is to be stored in the opening storage portion.

The used needle, stored in the piston rod, prevents an incident from occurring and is safely disposed.

The disposable injector syringe further includes, inside the opening storage portion, a closing diaphragm having crossing notches.

The needle is pushed against the closing diaphragm and stored in the piston rod. The needle cannot be pulled out, and the reuse of the used syringe is completely avoided.

The disposable injector syringe includes a reverse-movement prevention stopper ring in the vicinity of the opening of the cylindrical barrel of the syringe body, and a large number of reverse-movement prevention projections arranged on the circumferential portion of the piston rod in the midway position of the piston rod. The reverse-movement prevention projections are stopped by the reverse-movement prevention stopper ring.

Since the reverse-movement prevention projections are stopped by the reverse-movement prevention stopper ring, the piston, once pushed in, cannot be pulled out. The used disposable injector syringe cannot be used again. This arrangement functions as the restricting means for restricting the movement of the piston.

The disposable injector syringe includes a slide cover slidably movable along the cylindrical barrel of the syringe body.

When the slide cover is slid as necessary, the slide cover serves as a needle cover, thereby protecting users from being injured by the needle.

The disposable injector syringe includes a liquid-prefilled flexible plastic pouch arranged in the cylindrical barrel, and a proximal sharp end of the needle is projected in the cylindrical barrel.

The liquid medicine is injected when the piston is pushed breaking the pouch behind the needle.

### Brief Description of the Drawings

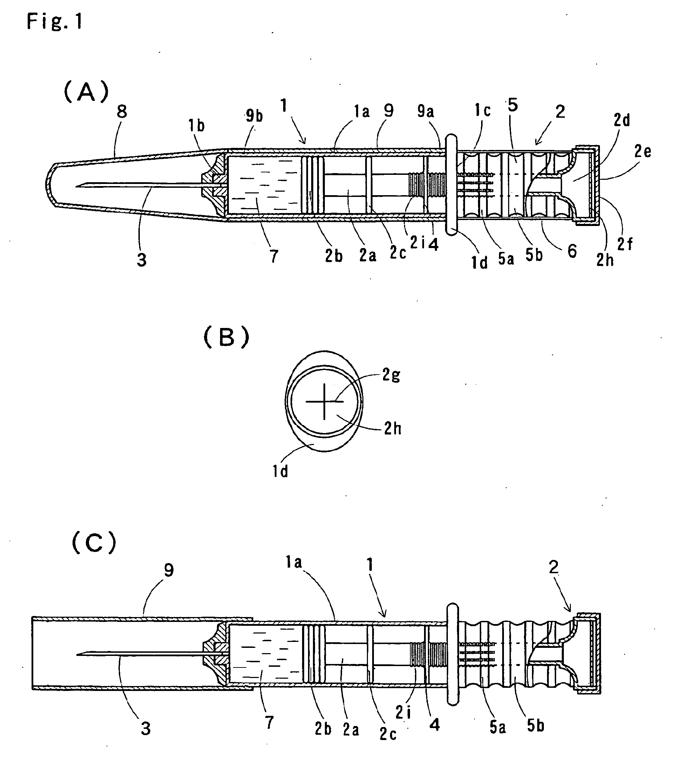

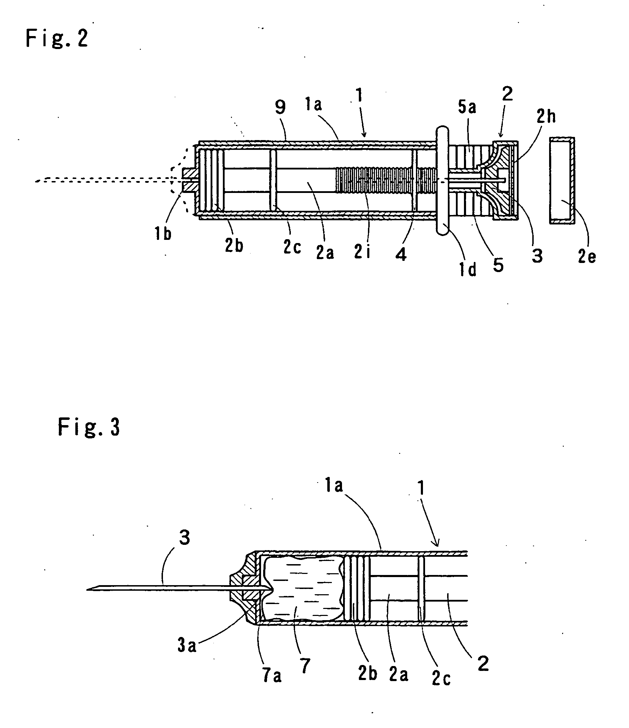

Fig. 1(A) is a partially sectional view of an disposable injector syringe in the storage state thereof in accordance with the present invention, Fig. 1(B) is a right end view of the disposable injector syringe with a cap removed, and Fig. 1(C) is a partially sectional view of the disposable injector syringe in a state prior to use. Fig. 2 is a partially sectional view of the disposable injector syringe with a needle stored in a piston after use, and Fig. 3 is a partially sectional view of a disposable injector syringe in accordance with another embodiment.

### Preferable Embodiment of the Invention

Disposable injector syringes in accordance with preferred embodiments of the present invention are described below with reference to the drawings. Fig. 1(A) is a partially sectional view of an disposable injector syringe in the storage state thereof in accordance with the present invention, Fig. 1(B) is a right end view of the disposable injector syringe with a cap removed, and Fig. 1(C) is a partially sectional view of the disposable injector syringe in a state prior to use. Fig. 2 is a partially sectional view of the disposable injector syringe with a needle stored in a piston after use.

As shown, a syringe body 1 includes a needle mounting portion 1b at one end of a cylindrical barrel 1a, and an opening portion 1c, at the other end of the cylindrical barrel 1a, for receiving a piston to be discussed later. A rounded flange-like finger grip 1d is arranged on the circumference of the opening portion 1c. The piston 2 is inserted into or removed from the cylindrical barrel 1a. The piston 2 includes a piston head 2b arranged at one end of a piston rod 2a having a hollow portion, and a stability guide ring 2c supported on the piston rod 2a slightly away from the piston head 2b. The piston 2 includes, at the other end of the piston rod 2a, an opening storage portion 2d that communicates with the hollow portion. The opening storage portion 2d is closed with a cap 2e. The cap 2e also functions as a finger push-in portion 2f.
A closing diaphragm 2h having crossing notches 2g is arranged within the opening storage portion 2d as shown in Fig. 1(B).
A needle 3 is mounted on the needle mounting portion 1b of the syringe body 1. Any known method, such as fitting, screwing, may be used to mount the needle.

A reverse-movement stopper ring 4 is arranged in the vicinity of the end of the opening portion of the syringe body 1. The reverse-movement stopper ring 4 stops a large number of reverse-movement stopper projections 2i in the intermediate portion of the piston rod 2a so that the piston 2 may not move in the direction of coming off.

A bellows 5 covers the piston rod 2a in the area thereof between the finger push-in portion 2f of the piston 2 and the finger grip 1d. The bellows 5 is constructed of a circumferential portion 5a having a linear width and a trough 5b, both portions alternating with each other. With the finger push-in portion 2f pushed, the trough portions 5b are folded and shrunk. The fixing sheet 6 is glued the circumference of the bellows 5 to restrain the piston 2 from undesirable moving. The fixing sheet 6 is glued using a peelable adhesive agent so that the fixing sheet 6 is easily peeled later. With this arrangement, no pre-filled liquid medicine is prevented from leaking, and the fixing sheet 6 is quickly peeled off before use. The means restraining the movement of the piston 2 is not limited to the fixing sheet 6. Any other known means may be adopted. For example, the engagement of the reverse-movement stopper ring 4 and the reverse-movement stopper projections 2i may also function as retaining means for restraining the movement of the piston 2.

The syringe body is prefilled with a required amount of a liquid medicine 7. Specifications, such as the name of the liquid medicine, the amount of the liquid medicine, and other items may be written and displayed on the cylindrical barrel 1a of the syringe body 1 to help the user easily identify the liquid medicine.

A needle cover 8 is detachably mounted on the end of the syringe body 1 to cover the needle 3. A slide cover 9 is slidably mounted along the cylindrical barrel of the syringe body, and is fixed by locking ribs 9a and 9b. The slide cover, if pulled forward, functions as the needle cover 8, and is arranged as necessary.

The disposable injector syringe thus constructed is used as described below. The disposable injector syringe in the state prefilled with a liquid medicine (Fig. 1(A)), functioning as an ampule, is purchased and stored. Each syringe is labeled with the name and amount of the liquid medicine, etc. Healthcare professionals are thus free from a job of sucking a liquid medicine from an ampule into the syringe. If the syringe is left, the users can avoid mixing up one from another because the name of the liquid medicine is labeled. Since a suction operation from the ampule is eliminated, the user can quickly handle the syringe in emergency situations for example in an ambulance car that could be jolting. A plurality of liquid medicines are used, mixed-up of the liquid medicines is avoided. The piston 2, secured by the fixing sheet 6 glued onto the circumference of the bellows 5, is not moved in a careless manner.

During use, the user verifies the name of the liquid medicine, peels the fixing sheet 6 off, and removes the needle cover 8. The syringe is now ready for use. If the handling of the syringe is temporarily suspended, the slide cover 9 is slid to cover the needle 3 (Fig. 1(C)). In this way, an incident that could be caused by the needle is controlled.

If the user pushes the finger push-in portion 2f with the finger grip 1d gripped by fingers, the bellows 5 shrinks, moving the piston 2, and injecting the liquid medicine 7. Since many reverse-movement stopper projections 2i arranged on the piston rod 2a are stopped by the reverse-movement stopper ring 4, the piston 2 cannot be withdrawn, and the syringe cannot be reused.

After use, as shown in Fig. 2, the needle 3 is detached from the syringe, and the cap 2e is removed from the piston 2. The needle 3 is then pushed in against the closing diaphragm 2h. In this way, the needle 3 is received in the hollow portion of the piston rod 2a and the opening storage portion 2d. Since the crossing notches 2g are arranged in the closing diaphragm 2h; the needle 3 cannot be easily taken out. This arrangement prevents human bodies from being injured by the needle and protects them from a secondary infection by the needle. This arrangement also prevents reuse and mal-intentional use of the needle.

Fig. 3 illustrates another embodiment of the present invention. In the preceding embodiment, the cylindrical barrel 1a is prefilled with the liquid medicine 7. In accordance with this embodiment, a flexible plastic pouch 7a is prefilled with a predetermined amount of liquid medicine 7 as shown in Fig. 3, and the flexible plastic pouch 7a is stored in the cylindrical barrel 1a. The needle 3 has a sharp proximal end 3a projected into the cylindrical barrel 1a. With the piston 2 moved forward, the flexible plastic pouch 7a prefilled with the liquid medicine is broken by the proximal end 3a of the needle 3 so that the liquid medicine 7 is ready to be injected. The remaining structure and the operation of the disposable injector syringe of this embodiment remain unchanged from those of the preceding embodiment.

### Industrial Applicability

As described above, the disposable injector syringe of the present invention prefilled with the predetermined amount of liquid medicine provides excellent advantages. For example time required to transfer the liquid medicine from an ampule to the syringe is saved. Errors and injuries, that could take place in the transfer job, are thus precluded.

Since the piston rod is covered with the bellows and the piston rod is restrained by gluing the fixing sheet on the bellows, the prefilled syringe is stored in the simple structure. The piston is prevented from being carelessly moved.

The piston has the hollow portion, and the storage portion is arranged at the proximal end of the piston rod to store the needle. This arrangement precludes incidents by the needle. After use, the syringe is safely disposed.

Since the movement of the piston is limited to one direction only, reuse of the syringe is impossible. This arrangement prevents infection by a reuse and mal-intentional use.

The plastic pouch, prefilled with the liquid medicine, stored in the cylindrical barrel provides excellent sterilization property to the liquid medicine. Manufacturing process, other than a step of filling the pouch with the liquid medicine, is performed using the same manufacturing line as the disposable injector syringe of the preceding embodiment. The present invention thus provides excellent mass productivity.

## Claims

1. A disposable injector syringe comprising a syringe body (1), the syringe body (1) including a cylindrical barrel (1a) having a needle mounting portion (1b) on one end thereof, and an opening portion (1c), on the other end thereof, for receiving a piston (2), a needle(3) to be mounted on the needle mounting portion (1b), the piston (2) to be inserted through the syringe body (1) through the opening portion (1c), and restricting means for restricting a movement of the piston (2), the restricting means restricting the movement of the piston (2) so that the cylindrical barrel(1a) is prefilled with a liquid medicine (7) and then closed by the piston (2),
**characterized in that**
the piston movement restricting means comprises a bellows (5), and a fixing sheet (6), the bellows (5) being composed of a circumferential portion (5a) having a predetermined width and a trough portion (5b), the two portions alternating with each other, the bellows(5) covering a piston rod (2a) of the piston (2), and the fixing sheet (6) being glued onto the circumferential portion (5a) of the bellows(5).

2. The disposable injector syringe according to claim 1, wherein the piston (2) has a stability guide ring (2c) supported on the piston rod (2a)

3. The disposable injector syringe according to claim 1 or 2, wherein the piston rod (2a) includes a hollow portion, an opening storage portion (2d) communicating with the hollow portion, and a cap (2e) closing the opening storage portion (2d), the needle(3) to be stored in the opening portion (1c).

4. The disposable injector syringe according to claim 3, further comprising, inside the opening storage portion (2d), a closing diaphragm (2h) having crossing notches (2g).

5. The disposable injector syringe according to any of the preceding claims, comprising a reverse-movement prevention stopper ring (4) in the vicinity of the opening of the cylindrical barrel (1a) of the syringe body (1), and a large number of reverse-movement prevention projections (2i) arranged on the circumferential portion of the piston rod (2a), wherein the reverse-movement prevention projections (2i) are stopped by the reverse-movement prevention stopper ring (4).

6. The disposable injector syringe according to any of the preceding claims, comprising a slide cover (9) slidably movable along the cylindrical barrel (1a) of the syringe body (1).

7. The disposable injector syringe according to any of the preceding claims, comprising a liquid-prefilled flexible plastic pouch (7a) arranged in the cylindrical barrel (1a), wherein a proximal sharp end (3a) of the needle (3) is projected in the cylindrical barrel (1a).

## Patentansprüche

1. Einweginjektionsspritze mit einem Spritzenkörper (1), wobei der Spritzenkörper (1) eine zylindrische Trommel (1a) mit einem Nadelanbringteilbereich (1b) an ihrem einen Ende und einem Öffnungsteilbereich (1c) an ihrem anderen Ende zum Aufnehmen eines Kolbens (2) enthält, einer an dem Nadelanbringteilbereich (1b) anzubringenden Nadel (3), dem durch den Spritzenkörper (1) durch den Öffnungsteilbereich (1c) einzufügenden Kolben (2) und einer Beschränkungseinrichtung zum Beschränken einer Bewegung des Kolbens (2), so dass die zylindrische Trommel (1a) mit flüssiger Medizin vorgefüllt und dann durch den Kolben (2) geschlossen wird,
**dadurch gekennzeichnet, dass**
die Kolbenbewegungs-Beschränkungseinrichtung einen Faltenbalg (5) und eine Fixierhülse (6) aufweist, wobei der Faltenbalg (5) aus einem Umfangsteilbereich (5a) mit einer vorbestimmten Breite und aus einem Muldenteilbereich (5b) zusammengesetzt ist, wobei die zwei Teilbereiche miteinander abwechseln, wobei der Faltenbalg (5) eine Kolbenstange (2a) des Kolbens (2) bedeckt und wobei die Fixierhülse (6) an den Umfangsteilbereich (5a) des Faltenbalgs (5) geklebt ist.

2. Einweginjektionsspritze nach Anspruch 1, wobei der Kolben (2) einen an der Kolbenstange (2a) gestützten Stabilitäts-Führungsring (2c) hat.

3. Einweginjektionsspritze nach Anspruch 1 oder 2, wobei die Kolbenstange (2a) einen hohlen Teilbereich, einen mit dem hohlen Teilbereich in Kommunikationsverbindung stehenden Öffnungslagerungsteilbereich (2d) und eine Kappe (2e), die den Öffnungslagerungsteilbereich (2d) schließt, hat, wobei die Nadel (3) in dem Öffnungsteilbereich (1c) zu lagern ist.

4. Einweginjektionsspritze nach Anspruch 3, die innerhalb des Öffnungslagerungsteilbereichs (2d) weiterhin eine Schließmembran (2h) mit sich kreuzenden Kerben (2g) hat.

5. Einweginjektionsspritze nach einem der vorangehenden Ansprüche, die einen Rückwärtsbewegungsverhinderungs-Stoppring (4) in der Nähe der Öffnung der zylindrischen Trommel (1a) des Spritzenkörpers (1) und eine große Anzahl von an dem Umfangsteilbereich der Kolbenstange (2a) angeordneten Rückwärtsbewegungsverhinderungs-Vorsprüngen (2i) aufweist, wobei die Rückwärtsbewegungsverhinderungs-Vorsprünge (2i) durch den Rückwärtsbewegungsverhinderungs-Stoppring (4) gestoppt werden.

6. Einweginjektionsspritze nach einem der vorangehenden Ansprüche, die eine Schiebeabdeckung (9) aufweist, die entlang der zylindrischen Trommel (1a) des Spritzenkörpers (1) schiebbar beweglich ist.

7. Einweginjektionsspritze nach einem der vorangehenden Ansprüche, die einen in der zylindrischen Trommel (1a) angeordneten, mit Flüssigkeit vorgefüllten, flexiblen Plastikbeutel (7a) aufweist, wobei ein nahes spitzes Ende (3a) der Nadel (3) in die zylindrische Trommel (1a) hervorsteht.

## Revendications

1. Seringue injecteur jetable comprenant un corps de seringue (1), le corps de seringue (1) incluant une colonne cylindrique (1a) ayant une portion de montage d'aiguille (1b) à une de ses extrémités, et une portion ouvrante (1c) à son autre extrémité, pour recevoir un piston (2), une aiguille (3) destinée à être montée sur la portion de montage d'aiguille (1b), le piston (2) destiné à être inséré à travers le corps de seringue (1) à travers la portion ouvrante (1c), et des moyens de restriction pour restreindre un mouvement du piston (2), les moyens de restriction restreignant le mouvement du piston (2) de sorte que la colonne cylindrique (1a) est pré-remplie d'un médicament liquide (7) et puis fermée par le piston (2),
**caractérisée en ce que**
les moyens de restriction du mouvement du piston comprennent un soufflet (5), et une feuille de fixation (6), le soufflet (5) étant composé d'une portion circonférentielle (5a) ayant une largeur prédéterminée et une portion d'auge (5b), les deux portions alternant l'une avec l'autre, le soufflet (5) couvrant une tige de piston (2a) du piston (2), et la feuille de fixation (6) étant collée sur la portion circonférentielle (5a) du soufflet (5).

2. Seringue injecteur jetable selon la revendication 1, dans laquelle le piston (2) a une bague de guidage de stabilité (2c) supportée sur la tige de piston (2a).

3. Seringue injecteur jetable selon la revendication 1 ou 2, dans laquelle la tige de piston (2a) inclut une portion creuse, une portion de stockage ouvrante (2d) communiquant avec la portion creuse, et un capuchon (2e) fermant la portion de stockage ouvrante (2d), l'aiguille (3) étant destinée à être stockée dans la portion ouvrante (1c).

4. Seringue injecteur jetable selon la revendication 3, comprenant également, à l'intérieur de la portion de stockage ouvrante (2d), une membrane de fermeture (2h) ayant des encoches croisées (2g).

5. Seringue injecteur jetable selon l'une quelconque des revendications précédentes, comprenant une bague de butée (4), empêchant un mouvement inverse, à proximité de l'ouverture de la colonne cylindrique (1a) du corps de seringue (1), et un grand nombre de saillies (2i) empêchant un mouvement inverse agencées sur la portion circonférentielle de la tige de piston (2a), dans laquelle les saillies (2i) empêchant un mouvement inverse sont stoppées par la bague de butée (4) empêchant un mouvement inverse.

6. Seringue injecteur jetable selon l'une quelconque des revendications précédentes, comprenant un couvercle coulissant (9) mobile en coulissement le long de la colonne cylindrique (1a) du corps de seringue (1).

7. Seringue injecteur jetable selon l'une quelconque des revendications précédentes, comprenant une poche (7a) en plastique souple pré-remplie de liquide agencée dans la colonne cylindrique (1a), dans laquelle une extrémité (3a) pointue proximale de l'aiguille (3) fait saillie dans la colonne cylindrique (1a).
